(19) Europäisches Patentamt
European Patent Office
Office européen des brevets

(11) **EP 3 870 060 B1**

(12) **FASCICULE DE BREVET EUROPEEN**

(45) Date de publication et mention de la délivrance du brevet:
**17.04.2024 Bulletin 2024/16**

(21) Numéro de dépôt: **19786351.7**

(22) Date de dépôt: **16.10.2019**

(51) Classification Internationale des Brevets (IPC):
**A61B 8/08** (2006.01)    **A61N 7/00** (2006.01)
**A61M 37/00** (2006.01)    **A61B 8/00** (2006.01)
**A61B 17/00** (2006.01)

(52) Classification Coopérative des Brevets (CPC):
**A61B 8/481; A61M 37/0092;** A61B 8/0808;
A61B 8/4488; A61N 2007/0039; A61N 2007/0086

(86) Numéro de dépôt international:
**PCT/EP2019/078098**

(87) Numéro de publication internationale:
**WO 2020/083725 (30.04.2020 Gazette 2020/18)**

(54) **SYSTÈME D'ANALYSE SPECTRALE ET DE DÉTERMINATION D'UN MARQUEUR PERMETTANT D'ASSURER LA SÉCURITÉ D'INTERVENTIONS D'ULTRASONS THÉRAPEUTIQUES**

SYSTEM ZUR SPEKTRALANALYSE UND ZUR BESTIMMUNG EINES MARKERS, DER ES ERMÖGLICHT, DIE SICHERHEIT VON THERAPEUTISCHEN ULTRASCHALLEINGRIFFEN SICHERZUSTELLEN

SYSTEM FOR SPECTRAL ANALYSIS AND DETERMINATION OF A MARKER MAKING IT POSSIBLE TO ENSURE THE SAFETY OF THERAPEUTIC ULTRASOUND INTERVENTIONS

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **24.10.2018 FR 1859826**

(43) Date de publication de la demande:
**01.09.2021 Bulletin 2021/35**

(73) Titulaire: **Commissariat à l'Energie Atomique et aux Energies Alternatives**
**75015 Paris (FR)**

(72) Inventeurs:
• **NOVELL, Anthony**
  **91300 MASSY (FR)**
• **SALLES KAMIMURA, Hermes**
  **NEW YORK, New York 10032 (US)**
• **LARRAT, Benoît**
  **75013 PARIS (FR)**

(74) Mandataire: **Atout PI Laplace**
**Immeuble "Visium"**
**22, avenue Aristide Briand**
**94117 Arcueil Cedex (FR)**

(56) Documents cités:
**US-A1- 2010 056 924    US-A1- 2013 006 106**

• **MCDANNOLD N ET AL: "Targeted disruption of the blood-brain barrier with focused ultrasound: association with cavitation activity; Cavitation activity and ultrasound-induced BBB disruption", PHYSICS IN MEDICINE AND BIOLOGY, INSTITUTE OF PHYSICS PUBLISHING, BRISTOL GB, vol. 51, no. 4, 21 février 2006 (2006-02-21), pages 793-807, XP020096136, ISSN: 0031-9155, DOI: 10.1088/0031-9155/51/4/003**

• **HERMES AS KAMIMURA ET AL: "Feedback control of microbubble cavitation for ultrasound-mediated blood-brain barrier disruption in non-human primates under magnetic resonance guidance", JOURNAL OF CEREBRAL BLOOD FLOW & METABOLISM, vol. 39, no. 7, 30 janvier 2018 (2018-01-30), pages 1191-1203, XP055602157, US ISSN: 0271-678X, DOI: 10.1177/0271678X17753514 cité dans la demande**

## Description

L'invention concerne un système correspondant à l'analyse spectrale de la réponse acoustique d'un tissu biologique et de détermination d'un marqueur de sécurité permettant d'assurer la sécurité d'interventions d'ultrasons thérapeutiques.

Le tissu biologique est un tissu biologique mou vascularisé quelconque, par exemple un tissu compris dans l'ensemble des tissus du cerveau, du foie, du coeur, des muscles, des seins, des reins, des yeux, de la thyroïde, de la prostate, de l'utérus, des tendons, du pancréas, de la peau, et de préférence un tissu cérébral.

Malgré l'accroissement du nombre de médicaments actifs et l'avènement des thérapies ciblées en cancérologie, les progrès thérapeutiques pour les maladies cérébrales (cancer inclus) restent encore modestes. L'un des obstacles majeurs réside dans l'absence de délivrance spécifique et contrôlée des molécules thérapeutiques au sein des tissus. En effet, les parois des vaisseaux sanguins cérébraux forment une barrière endothéliale très efficace appelée barrière hémato-encéphalique. Elle limite le passage de molécules du sang vers les cellules à traiter. Les méthodes actuelles pour administrer les agents thérapeutiques sont invasives, non localisées ou présentent un risque élevé pour le patient. En outre, la libre circulation des substances thérapeutiques dans l'organisme provoque des effets indésirables sur les tissus sains. L'acheminement efficace, spécifique et localisé de molécules thérapeutiques constitue donc un défi majeur. Depuis 2000, de nombreuses études ont démontré que les ultrasons focalisés peuvent être utilisés pour accomplir cette tâche. Combinés à l'injection par voie intraveineuse de microbulles de gaz, les ultrasons peuvent être utilisés afin d'induire une ouverture localisée et réversible des barrières biologiques. En effet, les forces mécaniques (i.e., micro-flux et oscillations) issues des interactions bulles-ultrasons (cavitation) fragilisent la barrière et favorisent le passage de molécules vers le tissu cérébral en général, et en particulier la région pathologique que l'on cherche à traiter si cette dernière est correctement visée par le faisceau d'ultrasons. La durée pouvant aller jusqu'à plusieurs heures, l'efficacité de l'ouverture de la barrière en termes de passage de larges molécules ainsi que la sécurité de la technique peuvent être contrôlées en modifiant les paramètres ultrasonores.

La « dose de cavitation » délivrée joue un rôle prépondérant dans l'efficacité et la sécurité de cette technologie. La pression acoustique au sein du tissu traité doit être suffisante pour entraîner une oscillation maîtrisée des microbulles (régime de cavitation stable) et engendrer une perméabilisation réversible et non lésionnelle des parois vasculaires.

En revanche, soumises à de trop fortes pressions acoustiques, les microbulles entrent alors dans un régime de cavitation inertielle impliquant des phénomènes physiques localement violents (i.e., onde de chocs, micro-jet, implosion locale de la bulle) pouvant conduire à une détérioration des tissus et en l'apparition d'effets secondaires graves (e.g., inflammation, hémorragie).

L'écart entre dose efficace et dose lésionnelle est faible et nécessite de développer de nouvelles méthodes précises de dosimétrie in situ. Le contrôle de la dose de cavitation en temps réel est donc un enjeu majeur à l'heure où cette technologie entame son passage en essais cliniques.

Dans le cadre de l'ouverture de la barrière hémato-encéphalique par ultrasons, l'objectif consiste à conserver un haut degré de cavitation stable (efficacité) pendant le traitement tout en maintenant la cavitation inertielle à un faible niveau (sécurité). Lors d'une thérapie par ultrasons transcrâniens, l'hétérogénéité du crâne peut avoir une influence indésirable sur l'efficacité et la sécurité de la technique.

En effet, l'épaisseur du crâne variant selon les zones, l'atténuation du faisceau ultrasonore s'en trouve modifiée. L'amplitude de l'onde ultrasonore peut aisément varier d'un facteur 2 d'un point à l'autre chez l'Homme. Pour les gros animaux et plus particulièrement les primates non humains, la présence de tissus (e.g,. des muscles) entre la peau et le crâne peut également modifier le faisceau ultrasonore.

Ainsi, lorsque les ultrasons sont transmis au travers une région plus épaisse qu'attendu, l'amplitude de l'onde ultrasonore dans la zone d'intérêt sera plus faible et le traitement potentiellement inefficace (i.e., la pression acoustique ne sera pas suffisante pour permettre une oscillation notoire des microbulles).

Au contraire, si la zone est moins épaisse, l'amplitude de l'onde ultrasonore sera sous-estimée et des problèmes de sécurité résultant de la cavitation inertielle des bulles pourront se produire.

La détection passive de cavitation peut être réalisée à l'aide de transducteurs ultrasonores disposés autour de la zone traitée. Ces capteurs permettent de mesurer la réponse fréquentielle issue des microbulles et ainsi de déterminer en temps réel le régime de cavitation induit, comme décrit dans l'article de Coussios et al., intitulé « Role of acoustic cavitation in the delivery and monitoring of cancer treatment by high-intensity focused ultrasound (HIFU) » et publié dans Int. J.Hyperthermia, March 2007, 23(2), pp.105-120.

Un défi actuel consiste à discriminer la réponse des microbulles de celle des tissus environnants puis d'assurer que cette réponse correspond à une cavitation stable. Par convention, la dose de cavitation stable est déterminée par le signal harmonique des microbulles :

$(n+1) f_0$ (avec $n \in \mathbb{N}^*$ et $f_0$ la fréquence d'émission des ultrasons) alors que la dose de cavitation inertielle correspond à la mesure du signal émis à toutes les fréquences (signal large bande), comme le décrit l'article de Konofagou et al. (2012), intitulé « Ultrasound-Induced Blood-Brain Barrier Opening" et publié dans Curr. Parma.Biotechnol. ;13(7), pp. 1332-1345.

Toutefois, ces marqueurs de cavitation manquent de robustesse et de sensibilité. Des évènements indésirables (e.g., oedèmes) peuvent survenir malgré les précautions. D'autres indicateurs plus fiables et reproductibles sont donc nécessaires.

De manière connue, les microbulles sont généralement composées d'un gaz lourd (perfluorocarbone, hexafluorure de soufre) afin de réduire leur cinétique de dissolution dans le sang et d'augmenter ainsi la durée de l'examen échographique qui atteint plusieurs minutes. Ce gaz est entouré d'une coque dont la fonction est de protéger la bulle. L'épaisseur de la paroi varie de quelques nm à plusieurs centaines de nm. Elle est généralement composée de protéines, de phospholipides, de surfactants ou de polymères.

De récents travaux in vitro, décrits dans l'article de Shekar et al. (2014), intitulé « The delayed onset of subharmonic and ultraharmonic émissions from a phospholipid contrast agent » et publié dans Ultrasound in Med.& Biol., 40(4), pp. 727-738, ont montré l'apparition d'oscillations spécifiques des microbulles lorsque ces dernières sont soumises à des conditions particulières. En effet, au cours du temps, le gaz contenu dans la bulle se diffuse dans le milieu environnant résultant sur un état de flambage de son enveloppe (e.g., excès de lipide sur l'enveloppe de la bulle). Cet état est associé à l'apparition de fréquences spécifiques (sub-harmonique et ultra-harmonique : $\frac{(2n+1)}{2}f_0$ avec $n \in \mathbb{N}$ et $f_0$ la fréquence d'émission des ultrasons) et peut conduire à la disparition de la microbulle.

Ce phénomène est accentué par l'application de longues séquences ultrasonores qui favorisent la diffusion du gaz, comme décrit dans l'article de O'Brien et al. (2013), intitulé « Surfactant shedding and gas diffusion during pulsed ultrasound through a microbubble contrast agent suspension » et publié dans J. Acoust. Soc. Am., 134, 1416-27, et le flambage de l'enveloppe de la microbulle comme décrit dans l'article de Kooiman et al. (2017), intitulé "Focal areas of increased lipid concentration on the coating of microbubbles during short tone-burst ultrasound insonification" et publié dans PLoS ONE 12(7):e0180747.

Cet état de déstabilisation des microbulles apparaît au cours de l'excitation ultrasonore et n'est pas à ce jour utilisé comme marqueur du risque d'effets indésirables lors d'une thérapie ultrasonore assistée par microbulles de gaz.

Il semblerait donc que l'on puisse en réalité définir trois régimes d'activité de microbulles : cavitation stable (rayonnement harmonique uniquement), cavitation inertielle (rayonnement harmonique, sub-harmonique, ultra-harmonique et large-bande) et un régime intermédiaire correspondant à une déstabilisation de la coque des microbulles (rayonnement harmonique, sub-harmonique et ultra-harmonique).

Aujourd'hui, les travaux mesurant l'activité de cavitation s'intéressent principalement aux composantes harmoniques et au spectre large-bande.

Actuellement, aucun consensus au niveau de la communauté scientifique n'est établi sur l'utilisation des composantes sub/ultra-harmoniques comme marqueur d'activité stable ou de cavitation inertielle, ainsi qu'en atteste l'article de Haqshenas et al. (2015), intitulé « Multi-resolution analysis of passive cavitation détecter signals, et publié dans Journal of Physics, doi :10.1088/1742-6596/581/1/012004.

Afin d'éviter les effets délétères liés à l'utilisation d'une trop forte pression acoustique, les travaux actuels déterminent un seuil de cavitation « tolérable » basé sur l'exploitation du signal large bande ou sur l'analyse d'une ou plusieurs composantes fréquentielles spécifiques (e.g., harmoniques, sub-harmonique, ultra-harmoniques). Parmi ces travaux actuels, peuvent être cités les documents suivants :

- l'article de O'Reilly et al. (2012), intitulé « Blood-Brain Barrier : Real-time Feedback-controlled Focused Ultrasound Disruption by Using an Acoustic Emissions-based Controller », et publié dans Radiology, 263(1), pp. 96-106 et la demande de brevet US 2013/006106A1 ;
- l'article de Tsai et al. (2016), intitulé "Real-time monitoring of focused ultrasound bloodbrain barrier opening via subharmonic acoustic émission détection: implementation of confocal dual-frequency piezoelectric transducers", et publié dans Phys.Med. Biol., 61, pp. 2926-2946 ;
- l'article de Kamimura et al. (2018), intitulé "Feedback control of microbubble cavitation for ultrasound-mediated blood-brain barrier disruption in non human primates under magnetic résonance guidance", et publié dans J. Cereb. Blood Flow Metab. 1 :271678X17753514 ;
- la demande de brevet WO2012042423 A1 ; et
- la demande de brevet WO2008062342 A3.

Qu'ils traitent l'information en temps-réel ou pas, avec une rétroaction ou pas sur le tir ultrasonore en cours, et qu'ils s'intéressent au spectre de rayonnement absolu ou relatif, ces travaux actuels ont comme point commun de calculer des doses de cavitation tir après tir, une valeur pour chaque tir. Ces marqueurs de sécurité souffrent d'un manque de sensibilité et n'empêchent pas dans certains cas l'apparition d'effets délétères.

Le problème technique est de fournir un marqueur de sécurité plus sensible, plus robuste et plus fiable qui permette d'éviter l'apparition d'effets délétères sur un tissu soumis à une exposition d'ultrasons thérapeutiques, et de fournir un procédé permettant de déterminer ce marqueur thérapeutique.

A cet effet, l'invention a pour objet un procédé d'analyse spectrale et de détermination d'un marqueur de sécurité, représentatif d'un état de déstabilisation de microbulles contenues dans une zone d'un tissu biologique mou vascularisé, lesdites microbulles étant soumises à un signal d'excitation ultrasonore à une fréquence d'émission $f_0$ prédéterminée pour induire une ouverture localisée et réversible des barrières biologiques dans ladite zone, et ledit état de déstabilisation des microbulles étant dommageable pour le tissu biologique, et ledit signal d'excitation ultrasonore étant formé par une séquence ultrasonore composée d'un nombre entier prédéterminé Nb, supérieur ou égal à 1, de train(s) d'onde, appelés « tir(s) ». Le procédé de détection et de détermination d'un marqueur de sécurité est caractérisé en ce qu'après le déclenchement de chaque tir Bb, b compris entre 1 et Nb, un système d'analyse spectrale et de détermination d'un marqueur de sécurité :

- mesure régulièrement au cours du tir Bb en une série d'instants ta, l'évolution temporelle des raies spectrales correspondant aux fréquences subharmoniques et ultra-harmoniques du signal de réponse acoustique des microbulles reçu, le signal de réponse reçu étant détecté par un détecteur passif de cavitation ayant une bande passante de détection prédéterminée, et

- détermine en la quantifiant l'évolution temporelle sur les instants ta d'un marqueur de sécurité, défini à chaque instant ta par un nombre $MDD_a$ égal au rapport de la somme des aires des raies spectrales, mesurées à l'instant ta et correspondant aux fréquences subharmoniques et/ou ultra-harmoniques du signal reçu de réponse acoustique des microbulles, sur la somme des aires des raies spectrales, mesurées au premier instant t1 et correspondant aux fréquences subharmoniques et/ou ultra-harmoniques du signal de réponse acoustique des microbulles dans leur état initial.

Suivant des modes particuliers de réalisation, le procédé d'analyse spectrale et de détermination d'un marqueur de sécurité comprend l'une ou plusieurs des caractéristiques suivantes :

- la mesure à chaque instant ta des raies spectrales correspondant aux fréquences subharmoniques et/ou ultra-harmoniques du signal de réponse acoustique des microbulles reçu exploite le signal de réponse reçu dans une fenêtre d'observation wa contenant l'instant ta et incluse dans l'intervalle temporel de réception correspondant au tir considéré ;
- les fenêtres d'observation ou d'analyse wa sont adjacentes ou séparées ou se recouvrent deux à deux partiellement ;
- le procédé d'analyse spectrale et de détermination d'un marqueur de sécurité décrit ci-dessus comprend pour un tir donné Bb, b compris entre 1 et Nb, une première étape de mesure et de segmentation dans laquelle le signal de réponse acoustique des microbulles au train d'onde Bb, reçu et mesuré par le détecteur passif de cavitation, est décomposé suivant un nombre entier prédéterminé k, supérieur ou égal à 2, de fenêtres temporelles wa, a variant de 1 à k, d'égales durées qui permettent de déterminer l'évolution des composantes fréquentielles au cours du train d'onde ultrasonore Bb ;
- le nombre k de fenêtres et leurs tailles tw dépendent directement de la durée et de la fréquence d'excitation $f_0$ du tir ultrasonore Bb, la durée du tir ultrasonore étant comprise entre quelques microsecondes et plusieurs centaines de millisecondes ;
- la durée des fenêtres wa est comprise entre la durée de 8 cycles du signal d'excitation et la moitié de la durée d'un tir, et/ou le nombre k de fenêtres wa est supérieur ou égal à 2 et inférieur ou égal au huitième du produit de la durée d'un tir $T_B$ par la fréquence d'excitation ultrasonore $f_0$ ;
- le procédé d'analyse spectrale et de détermination d'un marqueur de sécurité décrit ci-dessus comprend pour un tir Bb donné, b compris entre 1 et Nb, une deuxième étape de calcul de spectres, exécutée après la première étape, dans laquelle pour chaque fenêtre wa du tir Bb, a variant de 1 à k, le système d'analyse spectrale et de détermination d'un marqueur de sécurité calcule le spectre fréquentiel de la portion du signal de réponse acoustique des microbulles au train d'onde Bb, contenue dans ladite fenêtre wa ;
- la méthode de calcul des spectres fréquentiels utilise une transformée de Fourier ;
- le procédé d'analyse spectrale et de détermination d'un marqueur de sécurité, défini ci-dessus, comprend pour un tir Bb donné, b compris entre 1 et Nb, une troisième étape de calcul de l'évolution au cours du tir d'un signal de cavitation s(a), exécutée après la deuxième étape, dans laquelle pour chaque fenêtre temporelle wa, a variant de 1 à k, le système d'analyse spectrale et de détermination d'un marqueur de sécurité calcule le signal de cavitation s(a) comme la somme des aires des raies spectrales, mesurées à l'instant ta et correspondant aux fréquences subharmoniques et/ou ultra-harmoniques du signal de réponse acoustique des microbulles ;
- le nombre des composantes ultra-harmoniques et/ou subharmoniques considéré pour le calcul du signal de cavitation s(a) dépend de la bande passante du ou des transducteur(s) utilisé(s) pour la détection de cavitation et formant le détecteur passif de cavitation ;
- l'amplitude des pics mesurés pour les composantes ultra-harmonique(s) et/ou subharmonique(s) comprises dans la bande passante du détecteur passif de cavitation sont utilisés en complément ou en remplacement dans le calcul du signal de cavitation s(a) ;
- le procédé d'analyse spectrale et de détermination d'un marqueur de sécurité, décrit ci-dessus, comprend pour un tir Bb donné, b compris entre 1 et Nb, une quatrième étape de calcul de l'évolution au cours du tir d'un marqueur de cavitation s(a), exécutée après la troisième étape (108), dans laquelle pour chaque fenêtre temporelle wa du tir Bb, a variant de 1 à k, le système d'analyse spectrale et de détermination d'un marqueur de sécurité calcule un marqueur de sécurité , appelé « dose de cavitation $MDD_a$ », défini par un nombre $MDD_a$, égal au rapport du signal de cavitation s(a) de la a-ième fenêtre wa sur le signal de cavitation s(1) de la première fenêtre temporelle w1, la dose de cavitation $MDD_a$ étant exprimé dans une échelle linéaire ou logarithmique ;

- le procédé d'analyse spectrale et de détermination d'un marqueur de sécurité, décrit ci-dessus, comprend pour un tir Bb donné, b compris entre 1 et Nb, une cinquième étape de calcul de l'évolution au cours du tir d'un premier paramètre d'alerte Al1(a) et/ou d'un deuxième paramètre d'alerte Al2(a), exécutée après la quatrième étape, dans laquelle le premier paramètre d'alerte Al1 est mis dans un état actif lorsque le marqueur de sécurité $MDD_a$ dépasse une première valeur seuil de sécurité Th1 prédéterminée, et le deuxième paramètre d'alerte Al2 est mis dans un état actif lorsque le nombre de fois nf où le marqueur de sécurité $MDD_a$ a dépassé la première valeur de seuil Th1 a dépassé une deuxième valeur de seuil Th2 prédéterminée ;

- le procédé d'analyse spectrale et de détermination d'un marqueur de sécurité, décrit ci-dessus, comprend pour un tir Bb donné, b compris entre 1 et Nb, une sixième étape, exécutée après la quatrième ou la cinquième étape, dans laquelle le système d'analyse spectral et de détermination d'un marqueur de sécurité transmet à un dispositif de commande et de contrôle qui intervient dans une boucle de rétroaction des paramètres de tir : les doses de cavitation $MDD_a$ évoluant au cours du tir fournies dans la quatrième étape, et/ou les états Al1(a), Al2(a) du premier paramètre d'alerte et/ou du deuxième paramètre d'alerte déterminés dans la cinquième étape.

L'invention a également pour objet un système d'analyse spectrale et de détermination d'un marqueur de sécurité, représentatif d'un état de déstabilisation de microbulles, contenues dans une zone d'un tissu biologique, lesdites bulles étant soumises à un signal d'excitation ultrasonore émis à une fréquence d'émission $f_0$ prédéterminée pour induire une ouverture localisée et réversible des barrières biologiques dans ladite zone du tissu, et ledit état de déstabilisation des microbulles étant dommageable pour le tissu biologique, ledit signal d'excitation ultrasonore étant formé par une séquence ultrasonore composée d'un nombre entier prédéterminé Nb, supérieur ou égal à 1 de train(s) d'onde, appelés « tir(s) ». Le système d'analyse spectrale et de détermination d'un marqueur de sécurité est caractérisé en ce qu'il est configuré pour, après le déclenchement de chaque tir Bb, b compris entre 1 et Nb :

- mesurer régulièrement au cours du tir Bb en une série d'instants ta, l'évolution temporelle des raies spectrales correspondant aux fréquences subharmoniques et/ou ultra-harmoniques d'un signal de réponse acoustique des microbulles reçu, le signal de réponse reçu étant détecté par un détecteur passif de cavitation ayant une bande passante de détection prédéterminée, et

- déterminer en la quantifiant l'évolution temporelle sur les instants ta de la série temporelle un marqueur de sécurité, défini à chaque instant ta par un nombre MDDa égal au rapport de la somme des aires des raies spectrales, mesurées à l'instant ta et correspondant aux fréquences subharmoniques et/ou ultra-harmoniques du signal reçu de réponse acoustique des microbulles, sur la somme des aires des raies spectrales, mesurées au premier instant t1 et correspondant aux fréquences subharmoniques et/ou ultra-harmoniques du signal de réponse acoustique des microbulles.

Suivant des modes particuliers de réalisation, le système d'analyse spectrale et de détermination d'un marqueur de sécurité comprend l'une ou plusieurs des caractéristiques suivantes :

- à chaque instant ta, la mesure des raies spectrales correspondant aux fréquences subharmoniques et/ou ultra-harmoniques du signal de réponse acoustique des microbulles reçu, exploite le signal de réponse reçu dans une fenêtre d'observation wa contenant l'instant ta et incluse dans l'intervalle temporel de réception correspondant au tir considéré ;

- les fenêtres d'observation ou d'analyse wa sont adjacentes ou séparées ou se recouvrent deux à deux partiellement et faiblement par leurs bords.

Dans un mode de réalisation à titre d'exemple, un produit ou programme d'ordinateur comprend un ensemble d'instructions configurées pour mettre en oeuvre le procédé d'analyse spectrale et de détermination d'un marqueur de sécurité, défini ci-dessus, lorsqu'elles sont chargées dans et exécutées par un calculateur ou plusieurs calculateurs implémentés dans le système d'analyse spectrale et de détermination d'un marqueur de sécurité défini ci-dessus.

L'invention a également pour objet un système d'assistance ultrasonore d'un traitement thérapeutique visant une zone d'un tissu biologique mou vascularisé contenant des microbulles, comprenant :

- un dispositif d'excitation et d'émission d'une séquence thérapeutique de tir(s) d'excitation à une fréquence d'émission prédéterminée $f_0$, focalisés sur la zone à traiter du tissu biologique,

- un capteur passif de cavitation pour détecter et mesurer la réponse des microbulles contenues dans la zone en réponse aux tirs de la séquence,

- un système d'analyse spectrale et de détermination d'un marqueur de sécurité, représentatif d'un état de déstabilisation des microbulles, décrit ci-dessus,
- un dispositif de commande et de contrôle des paramètres de tir(s) du dispositif d'excitation ultrasonore,

le capteur passif de cavitation, le système d'analyse spectrale et de détermination d'un marqueur de sécurité, le dispositif de commande et de contrôle et le dispositif d'excitation ultrasonore étant mis en série selon un chaîne de sorte à former une boucle rétroactive de sécurité.

L'invention sera mieux comprise à la lecture de la description de plusieurs formes de réalisation qui va suivre, donnée uniquement à titre d'exemple et faite en se référant aux dessins sur lesquels :

- la Figure 1 est une vue schématique d'un système d'assistance ultrasonore d'un traitement thérapeutique visant une zone d'un tissu biologique contenant des microbulles, un système d'analyse spectrale et de détermination d'un marqueur de sécurité étant intégré dans ledit système d'assistance ultrasonore ;
- la Figure 2 est une vue d'une séquence ultrasonore pour la thérapie avec microbulles de gaz (e.g., ouverture de la barrière hémato-encéphalique, sonoperméabilisation) ;
- la Figure 3 est une vue du contenu fréquentiel de la réponse des microbulles en réponse à l'application de séquences de tirs ultrasonores ;
- la Figure 4 est une vue du procédé d'analyse spectrale et de détermination d'un marqueur de sécurité, ledit procédé étant exécuté lors de chaque tir d'une séquence ;
- la Figure 5 est une vue de la décomposition en plusieurs fenêtres temporelles $w_a$, a variant de 1 à k, du signal de cavitation reçu, issu des microbulles pendant un tir ultrasonore ;
- la Figure 6 est une vue d'un exemple de réponses spectrales avant et après déstabilisation des microbulles au cours d'un tir d'excitation ultrasonore transmis à 0,5 MHz dans le cerveau de primate ;
- la Figure 7 est une vue de l'évolution de la dose de composantes ultra-harmoniques $(1,5f_0$ ; $2,5f_0$ ; $3,5f_0)$ issues du signal des microbulles (SonoVue) au cours d'un tir de 10 ms à 500 kHz appliqué dans le cerveau de primate, un premier exemple aboutissant à l'apparition d'une hémorragie et un deuxième exemple sans survenue d'hémorragie ;
- les Figures 8A et 8B sont des vues de mesure du signal ultra-harmonique au cours des séquences ultrasonores chez le primate dans un cas non hémorragique pour la Figure 8A et un cas hémorragique pour la Figure 8B.

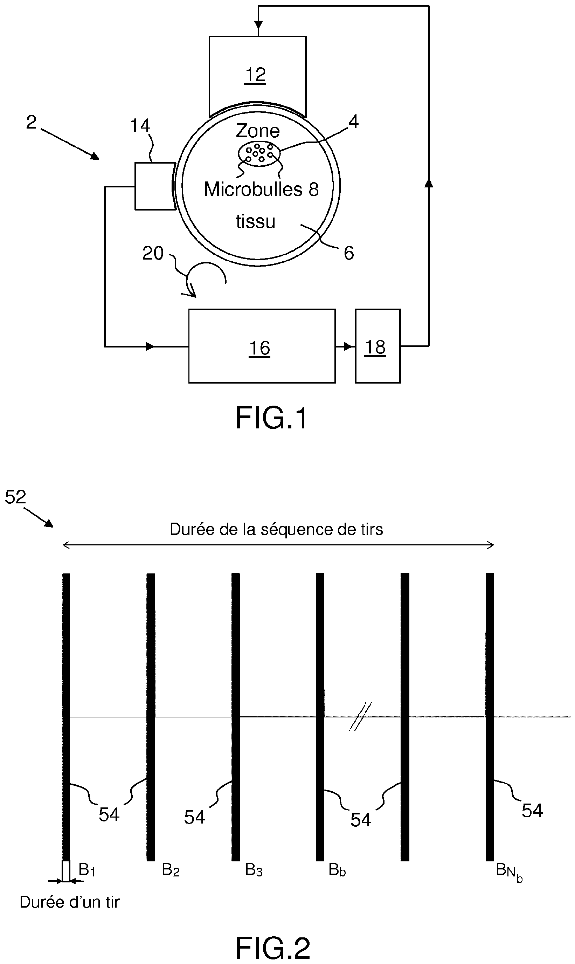

Suivant la Figure 1, un système d'assistance ultrasonore 2 d'un traitement thérapeutique visant une zone 4 d'un tissu biologique 6 contenant des microbulles 8 comprend :

- un dispositif 12 d'excitation et d'émission d'une séquence thérapeutique de trains d'onde d'excitation à une fréquence d'émission prédéterminée $f_0$, appelés « tirs », focalisés sur la zone 4 à traiter du tissu biologique 6 ;
- un capteur passif de cavitation 14, réalisé à l'aide d'un ou plusieurs transducteurs de réception, pour détecter et mesurer la réponse des microbulles contenues dans la zone 4 en réponse aux tirs de la séquence ;
- un système selon l'invention 16 d'analyse spectrale et de détermination d'un marqueur de sécurité, représentatif d'un état de déstabilisation des microbulles 8 ; et
- un dispositif 18 de commande et de contrôle des paramètres de tir(s) du dispositif d'excitation 12.

La fréquence d'excitation $f_0$ est choisie de sorte à permettre d'induire une ouverture localisée et réversible des barrières biologiques dans la zone traitée 4 du tissu 8.

Le capteur passif de cavitation 14 a une bande passante de réception qui dépend de la ou des bandes passantes des transducteurs électroacoustiques de réception.

Le système selon l'invention 16 d'analyse spectrale et de détermination d'un marqueur de sécurité est réalisé, par exemple, par un ou plusieurs calculateurs électroniques.

Le système selon l'invention 16 d'analyse spectrale et de détermination d'un marqueur de sécurité est configuré pour, après le déclenchement de chaque tir Bb d'une séquence d'un nombre Nb prédéterminé de tirs, Nb étant un nombre entier supérieur ou égal à 1 et b étant un indice d'ordre dans la séquence compris entre 1 et Nb :

- mesurer régulièrement au cours du tir Bb en une série d'instants ta, l'évolution temporelle des raies spectrales correspondant aux fréquences subharmoniques et ultra-harmoniques d'un signal de réponse acoustique des microbulles reçu, le signal de réponse reçu étant détecté par un détecteur passif de cavitation ayant une bande passante de détection prédéterminée, et
- déterminer en la quantifiant l'évolution temporelle sur les instants ta de la série temporelle un marqueur de sécurité, défini à chaque instant ta par un nombre $MDD_a$ égal au rapport de la somme des aires des raies spectrales, mesurées à l'instant ta et correspondant aux fréquences subharmoniques et/ou ultra-harmoniques du signal reçu de réponse acoustique des microbulles, sur la somme des aires des raies spectrales, mesurées au premier instant t1 et correspondant aux fréquences subharmoniques et/ou ultra-harmoniques du signal de réponse acoustique des microbulles.

Le dispositif 18 de commande et de contrôle des paramètres de tir(s) du dispositif d'excitation 12 est configuré pour recevoir en temps réel l'information du marqueur de sécurité et/ou des informations d'alerte et à partir de ces informations inhiber le dispositif d'excitation (arrêt du ou des tirs, modulation des paramètres de la séquence de tir(s)) lorsque la mise en sécurité du tissu l'exige.

Une boucle rétroactive de sécurité 20 automatique peut être mise en oeuvre par la mise en série selon une chaine du capteur de cavitation 14, du système d'analyse spectrale et de détermination d'un marqueur de sécurité 16, du dispositif de commande et de contrôle 18 des paramètres de tir(s), et du dispositif d'excitation et d'émission 12 d'une séquence thérapeutique de tir(s) ultrasonore(s), comme illustré sur la Figure 1.

Un signal d'excitation ultrasonore est formé par une séquence ultrasonore composée d'un nombre entier prédéterminé Nb, supérieur ou égal à 1, de train(s) d'onde, appelés « tir(s) »; et

Suivant la Figure 2, la séquence ultrasonore thérapeutique 52 est composée de plusieurs trains d'onde 54 (en anglais « burst »), appelés par la suite « tirs », répétés et séparés par des temps de repos. Typiquement, des tirs d'une durée comprise entre 3 et 10 ms sont répétés à une fréquence de répétition, de valeur comprise typiquement entre 5 et 10Hz, pendant une durée de séquence ou d'exposition de 30 secondes à 10 minutes.

Comme il a été déjà décrit en préambule, actuellement, la détection de cavitation (stable ou inertielle) est réalisée sur l'analyse spectrale de la réponse acoustique globale des microbulles à un train d'onde, c'est à dire la moyenne sur la durée totale de chaque tir. Comme mentionné précédemment, les microbulles peuvent émettre des fréquences spécifiques (sub-harmoniques et ultra-harmoniques) liées à leur déstabilisation.

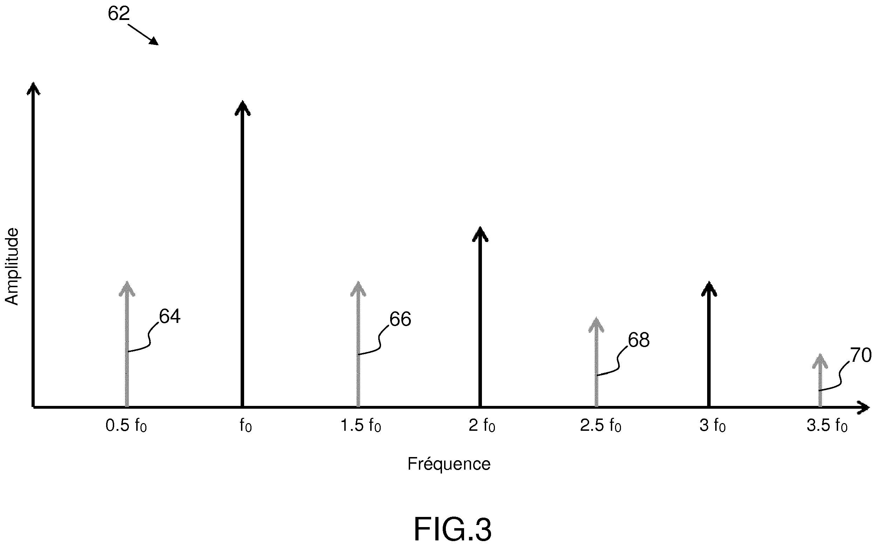

Suivant la Figure 3, l'apparition dans le contenu spectral 62 d'un tir pris dans sa globalité de raies à des fréquences subharmoniques et ultra-harmoniques est illustrée. Ici, une raie subharmonique 64 à $0,5\, f_0$ est observé et trois raies ultra-harmoniques 66, 68, 70, respectivement à $1,5\, f_0$ ; $2,5\, f_0$ ; $3,5\, f_0$ sont observées. L'apparition des raies subharmoniques et ultra-harmoniques est augmentée lorsque des tirs de longues durée, c'est-à-dire de plusieurs cycles d'onde à $f_0$ sont transmis.

Dans l'invention, les signaux de cavitation sont utilisés et exploités afin de déterminer un marqueur de sécurité en temps réel de la thérapie ultrasonore.

L'invention repose sur la mesure de l'évolution des fréquences subharmoniques et ultra-harmoniques au cours d'un tir ultrasonore (un train d'onde d'excitation émis) afin d'assurer la sécurité de la séquence thérapeutique. Les données ayant conduit à l'invention indiquent que l'apparition de raies à des fréquences subharmoniques et ultra-harmoniques dans le contenu spectral est un phénomène soudain qui se produit au cours du tir ultrasonore et qui perdure ensuite le temps de celui-ci.

La détection de ce phénomène peut être utilisée immédiatement pour arrêter le tir ultrasonore avant de reprendre le tir suivant une fois des nouvelles bulles intactes entrées dans le volume de tir. La séquence peut aussi être ajustée dynamiquement en terme d'amplitude ultrasonore et/ou de durée des tirs afin d'éviter la répétition du phénomène.

L'idée d'observer l'apparition de ce phénomène au cours du tir permet donc de définir précisément le temps à partir duquel ledit phénomène se produit et ainsi d'ajuster en conséquence la durée des tirs suivants de la séquence ultrasonore soit de façon fixe, soit préférentiellement de façon dynamique.

La mesure de ce phénomène au cours du tir suivant le procédé de l'invention est plus sensible que les outils de détection conventionnels basés sur l'analyse fréquentielle d'un tir complet.

Dans le cas de l'analyse conventionnelle des données mesurées par le capteur de cavitation, ces phénomènes d'apparition soudaine peuvent se produire sans être détectés. En effet, l'analyse conventionnelle revient à réaliser une moyenne du contenu fréquentiel sur le tir complet. Il devient évident que dans le cas où le phénomène apparaît à la fin du tir, le moyennage masquera les composantes subharmoniques et ultra-harmoniques qui n'apparaîtront pas (ou trop peu) lors de l'analyse fréquentielle.

En plus de ce phénomène de dilution du paramètre utile par moyennage, il faut noter que le suivi tir à tir observe des réalisations indépendantes sur des nuages de bulles différents à chaque tir. Les stratégies conventionnelles, actuellement proposées, qui analysent le signal de réponse sur le tir complet, utilisent comme référence un signal acquis avant injection des microbulles. Cette acquisition permet de prendre entre autre en considération le signal réfléchi par l'os et la propagation non linéaire des ultrasons dans le milieu. Toutefois, cette acquisition est réalisée plusieurs secondes à plusieurs minutes avant le traitement. Cette acquisition est contraignante car elle doit être répétée pour toutes les amplitudes ultrasonores potentiellement évaluées lors de l'injection des microbulles et les positions du transducteur pendant la thérapie. Elle nécessite une parfaite immobilisation du système ultrason et du patient pendant tout le traitement.

A contrario, dans l'invention, ce qui est observé est l'évolution du signal des mêmes bulles au sein du tir, c'est-à-dire la dynamique de leur déstabilisation sous ultrasons, en comparant les bulles à elles-mêmes en début de tir. La référence étant prise sur les même bulles quelques cycles auparavant, avec comme seule variable leur exposition récente aux ultrasons, il est logique d'exhiber des paramètres plus physiquement pertinents d'une telle analyse. Avec le procédé de l'invention, les difficultés évoquées précédemment, rencontrées avec les stratégies conventionnelles disparaissent, puisque le signal de référence est établi sur la première fenêtre w1 du tir pendant le traitement et l'application des ultrasons. Il en résulte un gain de temps et l'absence de perturbation.

De manière générale, un procédé d'analyse spectrale et de détermination d'un marqueur de sécurité selon l'invention est mis en oeuvre par le système 16 d'analyse spectrale et de détermination d'un marqueur de sécurité.

Le marqueur de sécurité selon l'invention est représentatif d'un état de déstabilisation de microbulles contenues dans la zone 4 du tissu biologique 6, lesdites microbulles 8 étant soumises au signal d'excitation ultrasonore à la fréquence d'émission $f_0$ prédéterminée pour induire une ouverture localisée et réversible des barrières biologiques dans la zone 4, et ledit état de déstabilisation des microbulles 8 étant dommageable pour le tissu biologique 6.

Le signal d'excitation ultrasonore est formé par une séquence ultrasonore composée d'un nombre entier prédéterminé Nb, supérieur ou égal à 1 de tirs.

Le procédé d'analyse spectrale et de détermination d'un marqueur de sécurité est caractérisé en ce qu'après le déclenchement de chaque tir Bb, b compris entre 1 et Nb, le système d'analyse spectrale et de détermination d'un marqueur de sécurité :

- mesure régulièrement au cours du tir Bb en une série d'un nombre entier prédéterminée k d'instants ta, a variant de 1 à k, l'évolution temporelle des raies spectrales correspondant aux fréquences subharmoniques et ultra-harmoniques du signal de réponse acoustique des microbulles reçu, le signal de réponse reçu étant détecté par un détecteur passif de cavitation ayant une bande passante de détection prédéterminée, et

- détermine en la quantifiant l'évolution temporelle sur les instants ta d'un marqueur de sécurité, défini à chaque instant ta par un nombre MDDa égal au rapport de la somme des aires des raies spectrales, mesurées à l'instant ta et correspondant aux fréquences subharmoniques et/ou ultra-harmoniques du signal reçu de réponse acoustique des microbulles, sur la somme des aires des raies spectrales, mesurées au premier instant t1 et correspondant aux fréquences subharmoniques et/ou ultra-harmoniques du signal de réponse acoustique des microbulles.

La mesure à chaque instant ta des raies spectrales correspondant aux fréquences subharmoniques et ultra-harmoniques du signal de réponse acoustique des microbulles reçu exploite le signal de réponse reçu dans une fenêtre d'observation wa contenant l'instant ta et incluse dans l'intervalle temporel de réception correspondant au tir considéré.

Les fenêtres d'observation ou d'analyse wa sont adjacentes ou séparées ou se recouvrent deux à deux partiellement et faiblement par leurs bords.

Il est à remarquer que la première fenêtre w1 qui sert de référence peut d'une part être légèrement décalée par rapport au début du tir pour attendre la stabilisation du signal et d'autre part la durée de la fenêtre w1 sur laquelle on calcule le spectre de référence peut être plus grande que la durée d'une fenêtre d'observation consécutive ou la durée de la somme de plusieurs fenêtres consécutives. Cela permet d'obtenir un spectre de référence moins bruité.

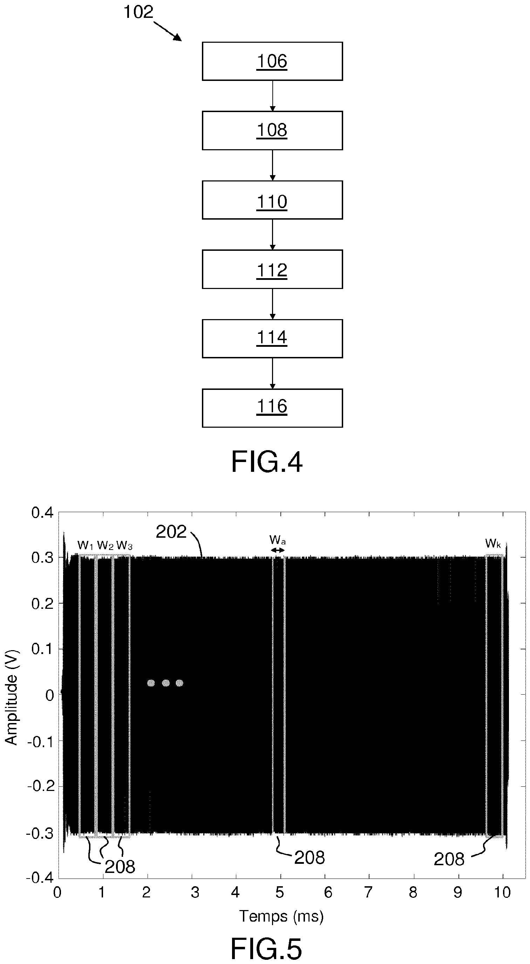

Suivant la Figure 4, un procédé 102 d'analyse spectrale et de détermination d'un marqueur de sécurité selon l'invention, exécuté lors de chaque tir Bb d'une séquence, b étant compris entre 1 et Nb, comprend un ensemble d'étapes 106, 108, 110, 112, 114 et 116.

Dans une première étape 106 de mesure et de segmentation, le signal de réponse temporelle des microbulles 8 au tir ultrasonore Bb, reçu et détecté au préalable par le détecteur passif de cavitation 14, est décomposé suivant un nombre entier prédéterminé k, supérieur ou égal à 2, de fenêtres temporelles wa, a variant de 1 à k, d'égales durées qui permettent de déterminer l'évolution des composantes fréquentielles au cours du train d'onde ultrasonore Bb.

Le nombre k de fenêtres wa et leurs tailles tw vont directement dépendre de la longueur $T_B$ et de la fréquence $f_0$ du tir ultrasonore utilisés pour la thérapie.

Typiquement, les travaux actuels montrent une ouverture réversible de la barrière pour des tirs ultrasonores dont la longueur varie de quelques microsecondes à plusieurs dizaines de millisecondes. D'un point de vue théorique, deux fenêtres temporelles de taille équivalentes suffisent pour réaliser la méthode. La première fenêtre temporelle w1 est alors utilisée comme référence, la seconde w2 permet d'observer l'évolution du signal reçu des microbulles.

Toutefois, si le nombre de fenêtres k n'est pas suffisamment grand, il sera impossible de déterminer avec précision, la précision étant déterminée par le nombre et la taille de la fenêtre, le temps d'apparition des fréquences subharmoniques et ultra-harmoniques, et donc d'ajuster efficacement les temps et amplitude de tir ultrasonore dans la séquence. D'autre part, l'utilisation de fenêtres temporelles trop longues induira un moyennage du contenu fréquentiel du signal reçu rendant difficile la détection des fréquences sub-harmoniques et ultra-harmoniques. Il est donc recommandé de subdiviser le signal récupéré en une multitude de fenêtres temporelles k consécutives, relativement courtes (si possible quelques dizaines de cycles ultrasonores) afin d'augmenter la sensibilité de la méthode.

Dans une deuxième étape 108 de calcul de spectres, exécutée après la première étape 106, pour chaque fenêtre temporelle wa (a, 1<a<k), le spectre fréquentiel de la portion du signal de réponse acoustique des microbulles au train d'onde Bb, contenue dans la fenêtre wa, est calculé afin d'effectuer une analyse du signal dans le domaine fréquentiel.

De manière préférée, la méthode de calcul des spectres fréquentiels utilise une transformée de Fourier.

Le nombre de cycle $n_c$ dans le signal temporel considéré doit être suffisamment grand ($n_c > 8$ cycles ultrasonores) afin d'éviter le recouvrement des composantes fréquentielles lors de l'analyse spectrale.

En résumé, et prenant en compte les exigences concernant la première étape 106 et la deuxième étape 108, la durée des fenêtres wa est comprise entre 8 cycles et la moitié de la durée $T_B$ d'un tir. Le nombre k de fenêtres wa est supérieur ou égal à 2 et inférieur ou égal au huitième du produit de la durée d'un tir $T_B$ par la fréquence d'excitation ultrasonore $f_0$.

Dans une troisième étape 110 de l'évolution au cours du tir Bb d'un signal de cavitation s(a), exécutée après la deuxième étape 108, pour chaque fenêtre temporelle wa, a variant de 1 à k, le signal de cavitation s(a) est calculé à partir de la représentation du signal dans le domaine fréquentiel, fournie dans la deuxième étape 108, comme la somme des aires des raies spectrales, mesurées à l'instant ta et correspondant aux fréquences subharmoniques et/ou ultra-harmoniques du signal de réponse acoustique des microbulles.

Le nombre de composantes ultra-harmoniques à considérer peut varier selon les dispositifs et la bande passante du ou des transducteur(s) utilisé(s) pour la détection de cavitation. C'est une question de rapport signal sur bruit de la détection des différentes composantes. L'analyse peut être réalisée sur une composante, de préférence $f_0/2$ ou $3 f_0/2$ qui sont les plus intensément rayonnées par les bulles, ou plusieurs composantes, par exemple $f_0/2$, $3 f_0/2$, $5 f_0/2$, $7 f_0/2$ et plus si le capteur passif de cavitation 14 le permet.

Il est à remarquer que l'amplitude des pics à ces composantes ultra-harmoniques et/ou subharmoniques peut également être considérée dans ce calcul du signal de cavitation, en complément ou remplacement de l'aire des raies spectrales sous la courbe de bandes spectrales. Les bandes spectrales $\Delta f$ utilisées pour calculer ces composantes fréquentielles dépendent de la fréquence d'émission $f_0$ et de la fréquence d'échantillonnage du dispositif de mesure et de la durée des fenêtres temporelles wa. Les travaux concernant cette invention montrent que la méthode peut être appliquée efficacement pour des bandes spectrales $\Delta f$ allant de $0,04 * f_0$ à $0,5 * f_0$ avec une préférence autour de $0,1* f_0$. La largeur de la bande spectrale doit être adaptée au nombre de cycle de la fenêtre temporelle afin de minimiser la mesure du bruit. Lorsque le nombre de cycles $n_c$ est grand, la bande spectrale $\Delta f$ doit être petite, et inversement lorsque le nombre de cycles $n_c$ est petit, la bande spectrale $\Delta f$ doit être grande.

Il est à remarquer qu'en variante on peut calculer séparément le signal de cavitation pour chacune des composantes ultra-harmoniques et/ou subharmoniques et considérer de manière associée des stratégies de rétroaction plus complexes dans un arbre de décision. Par exemple, selon la bande passante du capteur de cavitation et le bruit électronique ambiant (plus présent à basse fréquence), si l'amplitude de la composante à $f_0/2$ « explose », c'est à dire est excessive, mais que l'amplitude de la composante à $3 f_0/2$ reste admissible, alors dans une tentative de rétroaction l'amplitude du faisceau est diminuée sans le couper puis si l'amplitude de la composante à $3 f_0/2$ finit par « exploser » alors le tir est vraiment coupé.

Dans une quatrième étape 112 de calcul de l'évolution au cours du tir Bb d'un marqueur de cavitation s(a), exécutée après la troisième étape 110, pour chaque fenêtre wa du tir Bb, a variant de 1 à k, le système d'analyse spectrale et de détermination d'un marqueur de sécurité calcule un marqueur de sécurité $MDD_a$, appelé « dose de cavitation $MDD_a$ » (en anglais « Microbubble Destabilization Dose »), défini par un nombre $MDD_a$, égal au rapport du signal de cavitation s(a) de la a-ième fenêtre wa sur le signal de cavitation s(1) de la première fenêtre temporelle w1, soit $MDD_a = \frac{s(a)}{s(1)}$, la dose de cavitation MDDa étant exprimée dans une échelle linéaire ou logarithmique.

De manière générale, les fenêtres d'observation ou d'analyse wa sont adjacentes ou séparées ou se recouvrent deux à deux partiellement.

L'exécution des première, deuxième, troisième et quatrième peut être mise en oeuvre en enchainement en mode série ou en mode pipeline.

Par exemple, dans un premier mode de réalisation, l'analyse du signal de réponse temporelle est conduite sur des fenêtres temporelles adjacentes successives.

Dans un deuxième mode de réalisation, l'analyse du signal de réponse temporelle est conduite sur des fenêtres temporelles glissantes avec toujours une normalisation par rapport au signal de cavitation s(1) de la première fenêtre.

Dans une cinquième étape 114 de calcul de l'évolution au cours du tir Bb d'un premier paramètre d'alerte Al1 et/ou d'un deuxième paramètre d'alerte Al2, exécutée par le système d'analyse spectrale et de détermination d'un marqueur de sécurité après la quatrième étape 112, le premier paramètre d'alerte Al1 est mis dans un état actif lorsque le marqueur de sécurité MDDa dépasse une première valeur seuil de sécurité Th1 prédéterminée, et le deuxième paramètre d'alerte Al2 est mis dans un état actif lorsque le nombre de fois nf où le marqueur de sécurité MDDa a dépassé la première valeur de seuil Th1 a dépassé une deuxième valeur de seuil Th2 prédéterminée. Ce nombre de fois nf peut être comptabilisé soit pour des tirs consécutifs ou en variante pour des tirs non nécessairement consécutifs.

Dans une sixième étape 116, exécutée après la quatrième 112 ou la cinquième étape 115, le système d'analyse spectrale et de détermination d'un marqueur de sécurité transmet les données suivantes au dispositif de commande et de contrôle qui intervient dans une boucle de rétroaction sur les paramètres de tir :

- les doses de cavitation $MDD_a$ évoluant au cours du tir fournies dans la quatrième étape ; et/ou
- les états AI1(a), AI2(a) du premier paramètre d'alerte et/ou du deuxième paramètre d'alerte déterminés dans la cinquième étape.

Par exemple, si la dose de cavitation MDD$_a$ dépasse un premier seuil de sécurité Th1 qui peut varier entre 6 et 22 dB selon les données et leurs post traitements avec une valeur préférentielle autour de 8 dB, la séquence ultrasonore de tirs peut être soit ajustée en temps réel par une boucle de rétrocontrôle, les paramètres de contrôle étant compris parmi la diminution de la pression acoustique, la réduction de la durée de tirs, et éventuellement l'espacement des tirs, ou arrêtée pour éviter tout effet indésirable lié aux ultrasons. Il est aussi possible de définir un deuxième seuil de sécurité Th2 comme un nombre maximum de tirs tolérables pouvant dépasser la dose MDD seuil, i.e. le premier seuil de sécurité Th1, tirs consécutifs ou non consécutifs.

A ce jour, aucun procédé et système d'assistance ultrasonore d'un traitement thérapeutique utilisant le marqueur MDD$_a$ de l'invention pour contrôler la sécurité d'une séquence ultrasonore thérapeutique n'est connu. Actuellement, l'analyse fréquentielle du signal de cavitation est effectuée sur la globalité du signal de réponse émis par les microbulles en réponse à chaque tir.

La décomposition du signal de réception de réponse à un tir sur au moins deux fenêtres temporelles permet d'observer l'apparition des raies correspondantes à composantes subharmoniques et/ou ultra-harmoniques au cours du tir. Ce phénomène d'apparition ne peut être ni observé ni quantifié correctement sans décomposition du signal de cavitation reçu sur au moins deux fenêtres temporelles.

De manière avantageuse, le bio-marqueur de sécurité MDD$_a$ selon l'invention, ses méthodes de calcul ainsi que son utilisation dans le cadre d'une boucle de rétrocontrôle sur les propriétés acoustiques du faisceau de tir permettent d'éviter la déstabilisation de la bulle engendrée par la modification de son enveloppe et par la diffusion du gaz contenu initialement à l'intérieur de celle-ci, laquelle déstabilisation peut entraîner des effets physiques localement violents comme cause possible d'effets biologiques délétères.

De manière avantageuse encore et bien que la motivation majeure de l'invention soit l'amélioration de la sécurité du protocole de traitement, le fait d'éviter la déstabilisation des microbulles permet de les maintenir actives dans le flux sanguin plus longtemps et ainsi d'augmenter la durée totale pendant laquelle on peut solliciter lesdites microbulles pour ouvrir la barrière hémato-encéphalique BHE et ainsi d'augmenter l'efficacité du traitement.

L'utilisation du marqueur de sécurité MDD selon l'invention peut permettre d'allonger les tirs, augmenter l'amplitude du faisceau ou augmenter le rapport cyclique des tirs (en anglais « duty cycle ») tant que le seuil délétère établi, par exemple un seuil de 8 dB, n'est pas atteint en cours de tir. En garantissant une moindre déstabilisation des microbulles, il allonge leur temps de circulation dans le sang et donc leur temps total d'interaction possible avec les parois de vaisseaux. Le marqueur de sécurité MDD selon l'invention peut donc servir d'outil d'optimisation d'efficacité, cet outil pouvant être combiné à d'autres outils plus classiques comme celui de la dose de cavitation harmonique.

Suivant la Figure 5, un exemple d'un signal de réponse acoustique 202 d'un milieu formé de microbulles, de tissu osseux et de tissus mous à un train d'onde, i.e un tir, de 10 ms transmis chez le primate à une fréquence f$_0$ de 0,5 MHz. La réponse 202 des microbulles au tir ultrasonore a été décomposée en 75 fenêtres temporelles 208 équivalentes wa, a variant de 1 à 75, de 128 μs de durée.

Il est à remarquer que la première fenêtre w1 ne débute pas nécessairement au temps de début du signal de réponse acquis. Ici sur la Figure 5, un délai de 160 μs a été pris. Cela permet au signal électronique de mesure de se stabiliser et rend possible la comparaison avec les autres fenêtres.

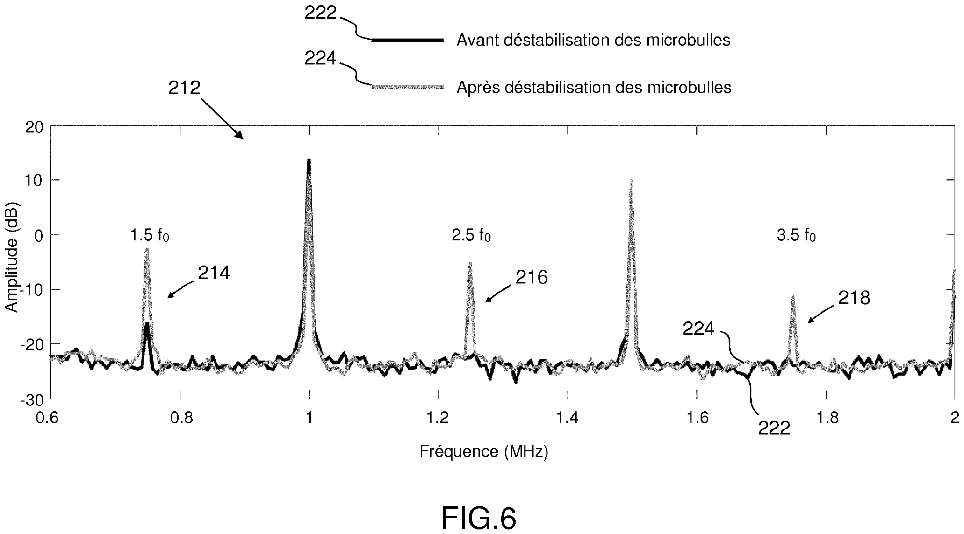

La Figure 6 montre un exemple de l'évolution 212 de la dose des composantes ultra-harmoniques 214, 216, 218 aux fréquences respectives 1,5f$_0$ ; 2,5f$_0$, ; 3,5f$_0$ entre une première réponse spectrale 222 en trait noir avant déstabilisation des microbulles et une deuxième réponse spectrale 224 en trait gris après déstabilisation des microbulles. Les première et deuxième réponses spectrales 222, 224 ont été mesurées dans deux fenêtres d'observations temporelles consécutives au cours d'un tir de 10 ms à 500 kHz appliqué dans le cerveau de primate pour l'ouverture de la barrière hémato-encéphalique et à partir du signal de réponse des microbulles reçu par le capteur passif de cavitation. Le signal de réponse reçu est mesuré avec un transducteur acoustique, formant le capteur de cavitation, dont la bande passante de réception est centrée à 1,5 MHz.

L'intervalle de temps séparant la mesure de la première réponse spectrale et la mesure de la deuxième réponse spectral est égale à la durée d'une fenêtre d'observation temporelle, soit 128 ps.

Le signal ultra-harmonique aux fréquences ultra-harmoniques 1,5f$_0$, 2,5f$_0$, 3,5f$_0$ correspondant à une déstabilisation des microbulles apparait nettement dans le deuxième spectre par rapport au premier spectre avec une augmentation nette du signal de cavitation s(a) associé.

Ainsi, à partir de l'analyse spectrale réalisée sur chaque fenêtre temporelle du signal de réponse au tir, la déstabilisation des microbulles peut être détectée.

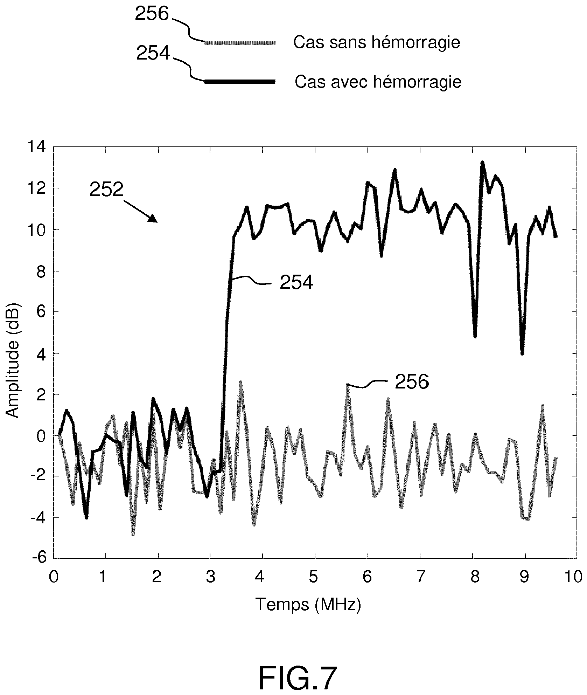

Suivant la Figure 7, l'évolution 252 de la dose de composantes ultra-harmoniques (1,5f$_0$ ; 2,5f$_0$ ; 3,5f$_0$) issues du signal des microbulles (SonoVue) au cours d'un tir de 10ms à 500 kHz appliqué dans le cerveau de primate est illustrée, un premier exemple aboutissant à l'apparition d'une hémorragie étant décrit par une première courbe 254 en trait noir, et un deuxième exemple sans survenue d'hémorragie étant décrit par une deuxième courbe 256 en trait gris.

Les première et deuxième courbes 254, 256 d'évolution démontrent que l'apparition des composantes ultra-harmoniques est un phénomène soudain se produisant à partir d'un certain temps en fonction des paramètres ultrasonores appliqués, du patient et du milieu soumis aux ultrasons. En effet, une excitation longue entraîne une oscillation répétée des bulles conduisant à leur potentielle déstabilisation à partir de plusieurs cycles d'excitation. Dans les conditions ultrasonores présentes, c'est-à-dire des trains d'ondes de 10ms à 500 kHz chez le primate et à 650 kHz chez le rat, les résultats d'étude concernant l'invention indiquent que le temps moyen d'apparition du phénomène est de 5,4 ms dans le cerveau de primate et de 3,2 ms dans le cerveau de rat. En pratique, un seuil de MDD de 8 dB a été considéré comme marqueur d'un évènement de déstabilisation des microbulles.

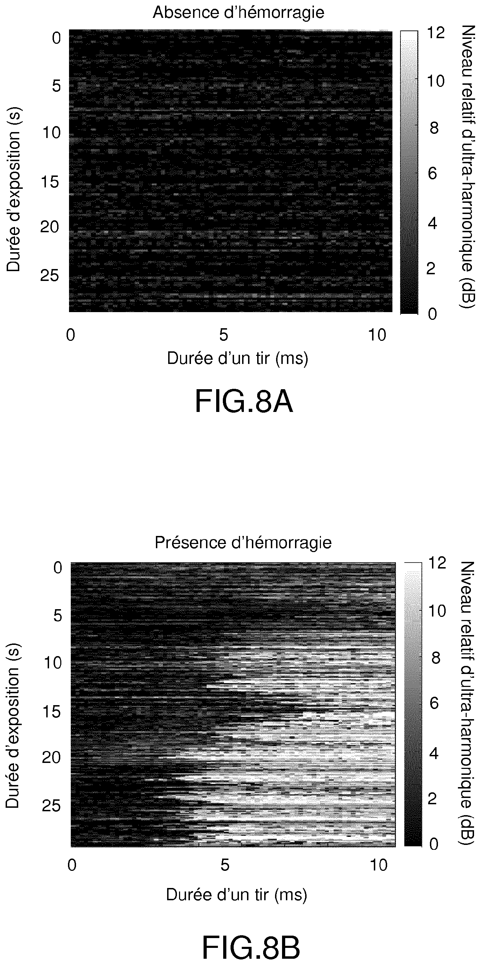

Les Figures 8A et 8B montrent des exemples d'un cas (Figure 8A) où aucun effet délétère n'a été visualisé et d'un cas (Figure 8B) où une hémorragie a été observée dans le cerveau du primate.

La déstabilisation des microbulles induite par la séquence ultrasonore est caractérisée par l'apparition de fréquences ultra-harmoniques, en clair sur la Figure 8B au cours du tir ultrasonore. La visualisation de ce phénomène spécifique est associée à l'observation d'une hémorragie chez le primate, confirmée par IRM (Imagerie par Résonance Magnétique).

Pour ces exemples, sur une période d'excitation ultrasonore de 30s, la fréquence d'apparition du phénomène est de 73,8% dans le cas hémorragique et de 0,7% dans le cas non hémorragique. L'apparition répétée de ce phénomène, c'est-à-dire plus de deux évènements pour des tirs consécutifs, est associée à la présence d'effets indésirables (oedèmes et hémorragies) chez l'animal.

Il est à noter que cet exemple se limite à l'étude des fréquences ultra-harmoniques (trois premières ultra-harmoniques) car la bande passante limitée en réception du capteur ultrasonore de cavitation ne permet pas une mesure fiable de la composante subharmonique dans cet exemple. Toutefois, l'utilisation d'un transducteur de détection de cavitation adapté à la mesure du signal subharmonique permet de réaliser une étude similaire sur la composante subharmonique.

Sur la Figure 8B, on peut mettre en évidence deux temps distincts : verticalement, le nombre de tirs avant que les bulles ne commencent à émettre des ultra-harmoniques pendant le tir. Puis horizontalement à partir de ce temps, la durée au début du tir avant qu'elles ne commencent à en émettre. Ces deux temps sont importants et renseignent sur la qualité des bulles dans le milieu.

## Revendications

1. Système d'analyse spectrale et de détermination d'un marqueur de sécurité (16), représentatif d'un état de déstabilisation de microbulles (8) contenues dans une zone (4) d'un tissu biologique (6) mou vascularisé, lesdites microbulles(8) étant soumises à un signal d'excitation ultrasonore à une fréquence d'émission $f_0$ prédéterminée pour induire une ouverture localisée et réversible des barrières biologiques dans ladite zone (4), et ledit état de déstabilisation des microbulles (8) étant dommageable pour le tissu biologique (6),

   ledit signal d'excitation ultrasonore étant formé par une séquence ultrasonore composée d'un nombre entier prédéterminé Nb, supérieur ou égal à 1 de train(s) d'onde, appelés « tir(s) » ; et le système d'analyse spectrale et de détermination d'un marqueur de sécurité étant **caractérisé en ce qu'**il est configuré pour, après le déclenchement de chaque tir Bb, b compris entre 1 et Nb :

      - recevoir un signal de réponse détecté par un détecteur passif de cavitation ayant une bande passante de détection prédéterminée,
      - mesurer (106, 108) régulièrement au cours du tir Bb en une série d'instants ta, l'évolution temporelle des raies spectrales correspondant aux fréquences subharmoniques et ultra-harmoniques du signal de réponse acoustique des microbulles reçu, et
      - déterminer (110, 112) en la quantifiant l'évolution temporelle sur les instants ta d'un marqueur de sécurité, défini à chaque instant ta par un nombre $MDD_a$ égal au rapport de la somme des aires des raies spectrales, mesurées à l'instant ta et correspondant aux fréquences subharmoniques et/ou ultra-harmoniques du signal reçu de réponse acoustique des microbulles, sur la somme des aires des raies spectrales, mesurées au premier instant t1 et correspondant aux fréquences subharmoniques et/ou ultra-harmoniques du signal de réponse acoustique des microbulles dans leur état initial.

2. Système d'analyse spectrale et de détermination d'un marqueur de sécurité selon la revendication 1, dans lequel la mesure à chaque instant ta des raies spectrales correspondant aux fréquences subharmoniques et/ou ultra-harmoniques du signal de réponse acoustique des microbulles reçu exploite le signal de réponse reçu dans une fenêtre d'observation wa contenant l'instant ta et incluse dans l'intervalle temporel de réception correspondant au tir considéré.

3. Système d'analyse spectrale et de détermination d'un marqueur de sécurité selon la revendication 2, dans lequel les fenêtres d'observation ou d'analyse wa sont adjacentes ou séparées ou se recouvrent deux à deux partiellement.

4. Système d'analyse spectrale et de détermination d'un marqueur de sécurité selon l'une quelconque des revendications 1 à 3, configuré pour un tir donné Bb, b compris entre 1 et Nb,
dans une première étape (104) de mesure et de segmentation, décomposer le signal de réponse acoustique des microbulles au train d'onde Bb, reçu et mesuré par le détecteur passif de cavitation, suivant un nombre entier prédéterminé k, supérieur ou égal à 2, de fenêtres temporelles wa, a variant de 1 à k, d'égales durées qui permettent de déterminer l'évolution des composantes fréquentielles au cours du train d'onde ultrasonore Bb.

5. Système d'analyse spectrale et de détermination d'un marqueur de sécurité selon la revendication 4, dans lequel

   le nombre k de fenêtres et leurs tailles tw dépendent directement de la durée et de la fréquence d'excitation $f_0$ du tir ultrasonore Bb, la durée du tir ultrasonore étant comprise entre quelques microsecondes et plusieurs centaines de millisecondes.

6. Système d'analyse spectrale et de détermination d'un marqueur de sécurité selon la revendication 4 ou la revendication 5, dans lequel

   la durée des fenêtres wa est comprise entre la durée de 8 cycles du signal d'excitation et la moitié de la durée d'un tir ; et/ou
   le nombre k de fenêtres wa est supérieur ou égal à 2 et inférieur ou égal au huitième du produit de la durée d'un tir $T_B$ par la fréquence d'excitation ultrasonore $f_0$.

7. Système d'analyse spectrale et de détermination d'un marqueur de sécurité selon l'une quelconque des revendications 4 à 6, configuré pour, un tir Bb étant donné avec b compris entre 1 et Nb,
dans une deuxième étape (106) de calcul de spectres, exécutée après la première étape (104), calculer pour chaque fenêtre wa du tir Bb, a variant de 1 à k, le spectre fréquentiel de la portion du signal de réponse acoustique des microbulles au train d'onde Bb, contenue dans ladite fenêtre wa.

8. Système d'analyse spectrale et de détermination d'un marqueur de sécurité selon la revendication 7, dans lequel le calcul des spectres fréquentiels utilise une transformée de Fourier.

9. Système d'analyse spectrale et de détermination d'un marqueur de sécurité selon l'une quelconque des revendications 7 à 8, configuré pour, un tir Bb étant donné avec b compris entre 1 et Nb,
dans une troisième étape (108) de calcul de l'évolution au cours du tir d'un signal de cavitation s(a), exécutée après la deuxième étape (106), calculer pour chaque fenêtre temporelle wa, a variant de 1 à k, le signal de cavitation s(a) comme la somme des aires des raies spectrales, mesurées à l'instant ta et correspondant aux fréquences subharmoniques et/ou ultra-harmoniques du signal de réponse acoustique des microbulles.

10. Système d'analyse spectrale et de détermination d'un marqueur de sécurité selon la revendication 9, dans lequel
le nombre des composantes ultra-harmoniques et/ou subharmoniques considéré pour le calcul du signal de cavitation s(a) dépend de la bande passante d'un ou plusieurs transducteur(s) utilisé(s) pour la détection de cavitation et formant le détecteur passif de cavitation.

11. Système d'analyse spectrale et de détermination d'un marqueur de sécurité selon l'une des revendications 9 à 10, dans lequel
l'amplitude des pics mesurés pour les composantes ultra-harmonique(s) et/ou subharmonique(s) comprises dans la bande passante du détecteur passif de cavitation sont utilisés en complément ou en remplacement dans le calcul du signal de cavitation s(a).

12. Système d'analyse spectrale et de détermination d'un marqueur de sécurité selon l'une quelconque des revendications 9 à 11, configuré pour, un tir Bb étant donné avec b compris entre 1 et Nb,
dans une quatrième étape (110) de calcul de l'évolution au cours du tir d'un signal de cavitation s(a), exécutée après la troisième étape (108), calculer pour chaque fenêtre temporelle wa du tir Bb, a variant de 1 à k, un marqueur de sécurité, appelé « dose de cavitation $MDD_a$ », défini par un nombre $MDD_a$, égal au rapport du signal de cavitation s(a) de la a-ième fenêtre wa sur le signal de cavitation s(1) de la première fenêtre temporelle w1, la dose de cavitation $MDD_a$ étant exprimé dans une échelle linéaire ou logarithmique.

13. Système d'analyse spectrale et de détermination d'un marqueur de sécurité selon la revendication 12, configuré pour, un tir Bb étant donné avec b compris entre 1 et Nb,

dans une cinquième étape (112) de calcul de l'évolution au cours du tir d'un premier paramètre d'alerte Al1(a) et/ou d'un deuxième paramètre d'alerte Al2(a), exécutée après la quatrième étape (110),

mettre le premier paramètre d'alerte Al1 dans un état actif lorsque le marqueur de sécurité MDD$_a$ dépasse une première valeur seuil de sécurité Th1 prédéterminée, et

mettre le deuxième paramètre d'alerte Al2 dans un état actif lorsque le nombre de fois nf où le marqueur de sécurité MDD$_a$ a dépassé la première valeur de seuil Th1 a dépassé une deuxième valeur de seuil Th2 prédéterminée.

14. Système d'analyse spectrale et de détermination d'un marqueur de sécurité selon l'une quelconque des revendications 12 à 13, configuré pour, un tir Bb étant donné avec b compris entre 1 et Nb,

dans une sixième étape (114), exécutée après la quatrième (110) ou la cinquième étape (112), transmettre à un dispositif de commande et de contrôle qui intervient dans une boucle de rétroaction des paramètres de tir :

- les doses de cavitation MDD$_a$ évoluant au cours du tir fournies dans la quatrième étape ; et/ou
- les états Al1(a), Al2(a) du premier paramètre d'alerte et/ou du deuxième paramètre d'alerte déterminés dans la cinquième étape.

15. Système d'assistance ultrasonore d'un traitement thérapeutique visant une zone (4) d'un tissu biologique (6) mou vascularisé contenant des microbulles (8), comprenant :

- un dispositif (12) d'excitation et d'émission d'une séquence thérapeutique de tir(s) d'excitation à une fréquence d'émission prédéterminée f$_0$, focalisés sur la zone (4) à traiter du tissu biologique (6),
- un capteur passif de cavitation (14) pour détecter et mesurer la réponse des microbulles (8) contenues dans la zone (4) en réponse aux tirs de la séquence,
- un système d'analyse spectrale et de détermination (16) d'un marqueur de sécurité, représentatif d'un état de déstabilisation des microbulles (8), défini selon l'une des revendications 1 à 14,
- un dispositif de commande et de contrôle (18) des paramètres de tir(s) du dispositif d'excitation ultrasonore (12),

le capteur passif de cavitation (14), le système d'analyse spectrale et de détermination d'un marqueur de sécurité (16), le dispositif de commande et de contrôle (18) et le dispositif d'excitation ultrasonore (12) étant mis en série selon un chaîne de sorte à former une boucle rétroactive de sécurité (20).

**Patentansprüche**

1. System zur Spektralanalyse und zur Bestimmung eines Sicherheitsmarkers (16), der für einen Destabilisierungszustand von Mikrobläschen (8) repräsentativ ist, die in einer Zone (4) eines weichen vaskularisierten biologischen Gewebes (6) enthalten sind, wobei die Mikrobläschen (8) einem Ultraschallanregungssignal mit einer vorbestimmten Emissionsfrequenz f$_0$ ausgesetzt sind, um eine lokalisierte und reversible Öffnung der biologischen Barrieren in der Zone (4) zu induzieren, und wobei der Destabilisierungszustand der Mikrobläschen (8) für das biologische Gewebe (6) schädlich ist,

wobei das Ultraschallanregungssignal durch eine Ultraschallsequenz gebildet wird, die aus einer vorbestimmten ganzen Zahl Nb größer als oder gleich 1 von einem Wellenzug/Wellenzügen, "Schuss/Schüssen" genannt, besteht; und wobei das System zur Spektralanalyse und zur Bestimmung eines Sicherheitsmarkers **dadurch gekennzeichnet ist, dass** es nach der Auslösung jedes Schusses Bb, wobei b zwischen 1 und Nb liegt, konfiguriert ist zum:

- Empfangen eines Antwortsignals, das von einem passiven Kavitationsdetektor mit einem vorbestimmten Detektionspassband detektiert wird,
- regelmäßigen Messen (106, 108), während des Schusses Bb zu einer Serie von Zeitpunkten ta, der zeitlichen Entwicklung der Spektrallinien entsprechend den subharmonischen und ultraharmonischen Frequenzen des empfangenen akustischen Antwortsignals der Mikrobläschen, und
- Bestimmen (110, 112), durch Quantifizieren, der zeitlichen Entwicklung über die Zeitpunkte ta eines Sicherheitsmarkers, der zu jedem Zeitpunkt ta durch eine Zahl MDD$_a$ definiert ist, die gleich dem Verhältnis der Summe der Flächen der Spektrallinien, die zum Zeitpunkt ta gemessen wurden und den subharmonischen und/oder ultraharmonischen Frequenzen des empfangenen akustischen Antwortsignals der Mikrobläschen entsprechen, zur Summe der Flächen der Spektrallinien ist, die zum ersten Zeitpunkt t1 gemessen wurden und den subharmonischen und/oder ultraharmonischen Frequenzen des akustischen Antwortsignals der Mikrobläschen in ihrem Anfangszustand entsprechen.

2. System zur Spektralanalyse und zur Bestimmung eines Sicherheitsmarkers nach Anspruch 1, wobei die Messung der Spektrallinien zu jedem Zeitpunkt ta, die den subharmonischen und/oder ultraharmonischen Frequenzen des empfangenen akustischen Antwortsignals der Mikrobläschen entsprechen, das empfangene Antwortsignal in einem Beobachtungsfenster wa auswertet, das den Zeitpunkt ta enthält und in dem Empfangszeitintervall inbegriffen ist, das dem betrachteten Schuss entspricht.

3. System zur Spektralanalyse und zur Bestimmung eines Sicherheitsmarkers nach Anspruch 2, wobei die Beobachtungs- oder Analysefenster wa benachbart oder getrennt sind oder sich paarweise teilweise überlappen.

4. System zur Spektralanalyse und zur Bestimmung eines Sicherheitsmarkers nach einem der Ansprüche 1 bis 3, konfiguriert zum, für einen gegebenen Schuss Bb, wobei b zwischen 1 und Nb liegt:
Zerlegen, in einem ersten Schritt (104) des Messens und Segmentierens, des akustischen Antwortsignals der Mikrobläschen auf den Wellenzug Bb, empfangen und gemessen von dem passiven Kavitationsdetektor, nach einer vorbestimmten ganzen Zahl k größer als oder gleich 2 von Zeitfenstern wa, wobei a von 1 bis k variiert, von gleicher Dauer, die es zulassen, die Entwicklung der Frequenzkomponenten im Verlauf des Ultraschallwellenzugs Bb zu bestimmen.

5. System zur Spektralanalyse und zur Bestimmung eines Sicherheitsmarkers nach Anspruch 4, wobei

die Anzahl k von Fenstern und ihre Größen tw direkt von der Dauer und der Anregungsfrequenz $f_0$ des Ultraschallschusses Bb abhängen, wobei die Dauer des Ultraschallschusses zwischen einigen Mikrosekunden und mehreren hundert Millisekunden liegt.

6. System zur Spektralanalyse und zur Bestimmung eines Sicherheitsmarkers nach Anspruch 4 oder Anspruch 5, wobei

die Dauer der Fenster wa zwischen der Dauer von 8 Zyklen des Anregungssignals und der Hälfte der Dauer eines Schusses liegt; und/oder die Anzahl k von Fenstern wa größer als oder gleich 2 und kleiner als ein oder gleich einem Achtel des Produkts aus der Dauer eines Schusses $T_B$ und der Ultraschallanregungsfrequenz $f_0$ ist.

7. System zur Spektralanalyse und zur Bestimmung eines Sicherheitsmarkers nach einem der Ansprüche 4 bis 6, konfiguriert zum, wenn ein Schuss Bb mit b zwischen 1 und Nb gegeben ist:
Berechnen in einem nach dem ersten Schritt (104) ausgeführten zweiten Schritt (106) des Berechnens von Spektren für jedes Fenster wa des Schusses Bb, wobei a von 1 bis k variiert, des Frequenzspektrums des Teils des akustischen Antwortsignals der Mikrobläschen auf den Wellenzug Bb, der in dem Fenster wa enthalten ist.

8. System zur Spektralanalyse und zur Bestimmung eines Sicherheitsmarkers nach Anspruch 7, wobei das Berechnen der Frequenzspektren eine Fourier-Transformation verwendet.

9. System zur Spektralanalyse und zur Bestimmung eines Sicherheitsmarkers nach einem der Ansprüche 7 bis 8, konfiguriert zum, wobei ein Schuss Bb gegeben ist, wobei b zwischen 1 und Nb liegt:
Berechnen, in einem nach dem zweiten Schritt (106) ausgeführten dritten Schritt (108) des Berechnens der Entwicklung, während des Schusses, eines Kavitationssignals s(a) für jedes Zeitfenster wa, wobei a von 1 bis k variiert, des Kavitationssignals s(a) als die Summe der Flächen der Spektrallinien, die zum Zeitpunkt ta gemessen werden und den subharmonischen und/oder ultraharmonischen Frequenzen des akustischen Antwortsignals der Mikrobläschen entsprechen.

10. System zur Spektralanalyse und zur Bestimmung eines Sicherheitsmarkers nach Anspruch 9, wobei die Anzahl der ultraharmonischen und/oder subharmonischen Komponenten, die bei der Berechnung des Kavitationssignals s(a) berücksichtigt werden, vom Passband eines oder mehrerer Wandler abhängt, die zur Kavitationsdetektion verwendet werden und den passiven Kavitationsdetektor bilden.

11. System zur Spektralanalyse und zur Bestimmung eines Sicherheitsmarkers nach einem der Ansprüche 9 bis 10, wobei
die Amplitude der gemessenen Peaks für die ultraharmonische(n) und/oder subharmonische(n) Komponente(n), die innerhalb des Passbandes des passiven Kavitationsdetektors liegen, zusätzlich oder alternativ bei der Berechnung des Kavitationssignals s(a) verwendet werden.

12. System zur Spektralanalyse und zur Bestimmung eines Sicherheitsmarkers nach einem der Ansprüche 9 bis 11, konfiguriert zum, wobei ein Schuss Bb gegeben ist, wobei b zwischen 1 und Nb liegt:
Berechnen, in einem nach dem dritten Schritt (108) ausgeführten vierten Schritt (110) des Berechnens der Entwicklung, während des Schusses, eines Kavitationssignals s(a) für jedes Zeitfenster wa des Schusses Bb, wobei a zwischen 1 und k variiert, eines Sicherheitsmarkers, genannt "Kavitationsdosis $MDD_a$", definiert durch eine Zahl $MDD_a$, die gleich dem Verhältnis des Kavitationssignals s(a) des a-ten Zeitfensters wa zum Kavitationssignal s(1) des ersten Zeitfensters w1 ist, wobei die Kavitationsdosis $MDD_a$ in einer linearen oder logarithmischen Skala ausgedrückt wird.

13. System zur Spektralanalyse und zur Bestimmung eines Sicherheitsmarkers nach Anspruch 12, konfiguriert zum, wobei ein Schuss Bb gegeben ist, wobei b zwischen 1 und Nb liegt:
in einem nach dem vierten Schritt (110) ausgeführten fünften Schritt (112) des Berechnens der Entwicklung, während des Schusses, eines ersten Warnparameters Al1(a) und/oder eines zweiten Warnparameters Al2(a):

Setzen des ersten Warnparameters Al1 in einen aktiven Zustand, wenn der Sicherheitsmarker $MDD_a$ einen ersten vorbestimmten Sicherheitsschwellenwert Th1 überschreitet, und
Setzen des zweiten Warnparameters Al2 in einen aktiven Zustand, wenn die Anzahl der Male nf, bei denen der Sicherheitsmarker $MDD_a$ den ersten Schwellenwert Th1 überschritten hat, einen zweiten vorbestimmten Schwellenwert Th2 überschritten hat.

14. System zur Spektralanalyse und zur Bestimmung eines Sicherheitsmarkers nach einem der Ansprüche 12 bis 13, konfiguriert zum, wobei ein Schuss Bb gegeben ist, wobei b zwischen 1 und Nb liegt:
Übertragen, in einem nach dem vierten (110) oder fünften (112) Schritt ausgeführten sechsten Schritt (114), zu einer Steuer- und Kontrollvorrichtung, die in eine Rückkopplungsschleife der Schussparameter eingreift:

- der sich während des Schusses entwickelnden Kavitationsdosen $MDD_a$, die im vierten Schritt geliefert werden; und/oder
- der Zustände Al1(a), Al2(a) des ersten Warnparameters und/oder des zweiten Warnparameters, die im fünften Schritt ermittelt werden.

15. System zur Ultraschallunterstützung einer therapeutischen Behandlung, die auf eine Zone (4) eines vaskularisierten weichen biologischen Gewebes (6) abzielt, das Mikrobläschen (8) enthält, das Folgendes umfasst:

- eine Vorrichtung (12) zum Anregen und Senden einer therapeutischen Sequenz von einem Anregungsschuss/Anregungsschüssen mit einer vorbestimmten Sendefrequenz $f_0$, die auf der zu behandelnden Zone (4) des biologischen Gewebes (6) lokalisiert sind,
- einen passiven Kavitationssensor (14) zum Detektieren und Messen der Antwort der in der Zone (4) enthaltenen Mikrobläschen (8) als Reaktion auf die Schüsse der Sequenz,
- ein System zur Spektralanalyse und zur Bestimmung eines Sicherheitsmarkers (16), der für einen Destabilisierungszustand der Mikrobläschen (8) repräsentativ ist, definiert nach einem der Ansprüche 1 bis 14,
- eine Steuer- und Kontrollvorrichtung (18) für die Schussparameter der Ultraschallanregungsvorrichtung (12),

wobei der passive Kavitationssensor (14), das System zur Spektralanalyse und zur Bestimmung eines Sicherheitsmarkers (16), die Steuer- und Kontrollvorrichtung (18) und die Ultraschallanregungsvorrichtung (12) gemäß einer Kette in Reihe geschaltet sind, so dass sie eine rückkoppelnde Sicherheitsschleife (20) bilden.

## Claims

1. A system for performing spectral analysis and determining a safety marker (16) that is representative of a state of destabilisation of microbubbles (8) contained in a region (4) of a soft vascularised biological tissue (6), said microbubbles (8) being subjected to an ultrasonic excitation signal at a predetermined emission frequency $f_0$ to induce localised and reversible opening of the biological barriers in said region (4), and said state of destabilisation of the microbubbles (8) being detrimental to the biological tissue (6),

said ultrasonic excitation signal being formed by an ultrasonic sequence composed of a predetermined integer number Nb, higher than or equal to 1, of wave train(s), called "shot(s)"; and the system for performing spectral analysis and determining a safety marker being **characterised in that** it is configured to, after each shot Bb is triggered, b being between 1 and Nb:

- receive a response signal detected by a passive cavitation detector having a predetermined detection passband,
- regularly measure (106, 108), during the shot Bb, at a series of times ta, the temporal variation in the spectral lines corresponding to the subharmonic and ultra-harmonic frequencies of the received acoustic-response signal of the microbubbles, and
- determine (110, 112), by quantifying it, the temporal variation, over the times ta, of a safety marker that is defined, at each time ta, by a number $MDD_a$ equal to the ratio of the sum of the surface areas of the spectral lines, measured at the time ta and corresponding to the subharmonic and/or ultra-harmonic frequencies of the received acoustic-response signal of the microbubbles, to the sum of the surface areas of the spectral lines, measured at the first time t1 and corresponding to the subharmonic and/or ultra-harmonic frequencies of the acoustic-response signal of the microbubbles in their initial state.

2. The system for performing spectral analysis and determining a safety marker according to claim 1, wherein the measurement, at each time ta, of the spectral lines corresponding to the subharmonic and/or ultra-harmonic frequencies of the received acoustic-response signal of the microbubbles exploits the response signal received in an observation window wa that contains the time ta and that is included in the reception time interval corresponding to the shot in question.

3. The system for performing spectral analysis and determining a safety marker according to claim 2, wherein the observation or analysis windows wa are adjacent or separate or partially overlap pairwise.

4. The system for performing spectral analysis and determining a safety marker according to any one of claims 1 to 3, configured for a given shot Bb, b being between 1 and Nb,
in a first measuring and segmenting step (104), to break up the acoustic-response signal of the microbubbles to the wave train Bb, said signal being received and measured by the passive cavitation detector, following a predetermined integer number k, k being higher than or equal to 2, of time windows wa, a varying from 1 to k, of equal durations that allow the variation in the frequency components during the ultrasonic wave train Bb to be determined.

5. The system for performing spectral analysis and determining a safety marker according to claim 4, wherein

the number k of windows and their sizes tw depend directly on the duration and the excitation frequency $f_0$ of the ultrasonic shot Bb,
the duration of the ultrasonic shot being comprised between a few microseconds and several hundred milliseconds.

6. The system for performing spectral analysis and determining a safety marker according to claim 4 or claim 5, wherein

the duration of the windows wa is comprised between the duration of 8 cycles of the excitation signal and half the duration of one shot; and/or the number k of windows wa is higher than or equal to 2 and lower than or equal to one eighth of the product of the duration of one shot $T_B$ multiplied by the ultrasonic excitation frequency $f_0$.

7. The system for performing spectral analysis and determining a safety marker according to any one of claims 4 to 6, configured to, a shot Bb being given with b between 1 and Nb,
in a spectra-computing second step (106), which is executed after the first step (104), compute, for each window wa of the shot Bb, a varying from 1 to k, the frequency spectrum of the portion of the acoustic-response signal of the microbubbles to the wave train Bb, that is contained in said window wa.

8. The system for performing spectral analysis and determining a safety marker according to claim 7, wherein the computation of the frequency spectra uses a Fourier transform.

9. The system for performing spectral analysis and determining a safety marker according to any one of claims 7 to 8, configured to, a shot Bb being given with b between 1 and Nb,
in a third step (108) of computing the variation, during the shot, in a cavitation signal s(a), which step is executed after the second step (106), compute, for each time window wa, a varying from 1 to k, the cavitation signal s(a) to be the sum of the surface areas of the spectral lines measured at the time ta and corresponding to the subharmonic and/or ultra-harmonic frequencies of the acoustic-response signal of the microbubbles.

10. The system for performing spectral analysis and determining a safety marker according to claim 9, wherein
the number of ultra-harmonic and/or subharmonic components considered in the computation of the cavitation signal s(a) depends on the passband of one or more transducer(s) used to detect the cavitation and that form(s) the passive cavitation detector.

11. The system for performing spectral analysis and determining a safety marker according to one of claims 9 to 10, wherein
the amplitude of the measured peaks for the ultra-harmonic and/or subharmonic component(s) comprised in the passband of the passive cavitation detector are added or substituted to each other in the computation of the cavitation signal s(a).

12. The system for performing spectral analysis and determining a safety marker according to any one of claims 9 to 11, configured to, a shot Bb being given with b comprised between 1 and Nb,

in a fourth step (110) of computing the variation, during the shot, in a cavitation signal s(a), which step is executed after the third step (108), compute, for each time window wa of the shot Bb, a varying from 1 to k, a safety marker called the "cavitation dose $MDD_a$", defined by a number $MDD_a$ equal to the ratio of the cavitation signal s(a) in the a-th window wa to the cavitation signal s(1) of the first time window w1, the cavitation dose $MDD_a$ being expressed on a linear or logarithmic scale.

13. The system for performing spectral analysis and determining a safety marker according to claim 12, configured to, a given Bb being given, b being between 1 and Nb,

in a fifth step (112) of computing the variation, during the shot, in a first warning parameter Al1(a) and/or in a second warning parameter Al2(a), which step is executed after the fourth step (110),

put the first warning parameter Al1 in an active state when the safety marker $MDD_a$ exceeds a first predetermined safety threshold value Th1, and

put the second warning parameter Al2 in an active state when the number of times nf the safety marker $MDD_a$ has exceeded the first threshold value Th1 has exceeded a second predetermined threshold value Th2.

14. The system for performing spectral analysis and determining a safety marker according to any one of claims 12 to 13, configured to, a shot Bb being given with b comprised between 1 and Nb,
in a sixth step (114), which step is executed after the fourth (110) or the fifth step (112), transmit, to a command and control device that intervenes in a feedback loop for the shot parameters:

- the cavitation doses $MDD_a$ delivered in the fourth step, said doses varying during the shot; and/or
- the states Al1(a), Al2(a) of the first warning parameter and/or of the second warning parameter, both determined in the fifth step.

15. A system for providing ultrasonic assistance to a therapeutic treatment targeting a region (4) of a soft vascularised biological tissue (6) containing microbubbles (8), comprising:

- a device (12) for exciting and emitting a therapeutic sequence of excitation shot(s) at a predetermined emission frequency $f_0$, which are focused on the region (4) to be treated of the biological tissue (6),
- a passive cavitation sensor (14) for detecting and measuring the response of the microbubbles (8) contained in the region (4) in response to the shots of the sequence,
- a system for performing spectral analysis and determining a safety marker (16) that is representative of a state of destabilisation of the microbubbles (8), defined in accordance with one of claims 1 to 14,
- a command and control device (18) for commanding and controlling parameters of the shot(s) of the ultrasonic exciting device (12),

the passive cavitation sensor (14), the system for performing spectral analysis and determining a safety marker (16), the command and control device (18) and the ultrasonic exciting device (12) being placed in series according to a chain so as to form a safety feedback loop (20).

Zone

Microbulles 8

tissu

FIG.1

Durée de la séquence de tirs

$B_1$   $B_2$   $B_3$   $B_b$   $B_{N_b}$

Durée d'un tir

FIG.2

FIG.3

102

106

108

110

112

114

116

## FIG.4

FIG.5

FIG.6

EP 3 870 060 B1

FIG.7

Absence d'hémorragie

FIG.8A

Présence d'hémorragie

FIG.8B

24

# RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- US 2013006106 A1
- WO 2012042423 A1
- WO 2008062342 A3

**Littérature non-brevet citée dans la description**

- **DE COUSSIOS et al.** Role of acoustic cavitation in the delivery and monitoring of cancer treatment by high-intensity focused ultrasound (HIFU). *Int. J.Hyperthermia,* Mars 2007, vol. 23 (2), 105-120
- **DE KONOFAGOU et al.** Ultrasound-Induced Blood-Brain Barrier Opening. *Curr. Parma.Biotechnol,* 2012, vol. 13 (7), 1332-1345
- **DE SHEKAR et al.** The delayed onset of subharmonic and ultraharmonic émissions from a phospholipid contrast agent. *Ultrasound in Med.& Biol.,* 2014, vol. 40 (4), 727-738
- **O'BRIEN et al.** Surfactant shedding and gas diffusion during pulsed ultrasound through a microbubble contrast agent suspension. *J. Acoust. Soc. Am.,* 2013, vol. 134, 1416-27
- **DE KOOIMAN et al.** Focal areas of increased lipid concentration on the coating of microbubbles during short tone-burst ultrasound insonification. *PLoS ONE,* 2017, vol. 12 (7), e0180747
- **DE HAQSHENAS et al.** Multi-resolution analysis of passive cavitation détecter signals. *Journal of Physics,* 2015
- **O'REILLY et al.** Blood-Brain Barrier : Real-time Feedback-controlled Focused Ultrasound Disruption by Using an Acoustic Emissions-based Controller. *Radiology,* 2012, vol. 263 (1), 96-106
- **DE TSAI et al.** Real-time monitoring of focused ultrasound bloodbrain barrier opening via subharmonic acoustic émission détection: implementation of confocal dual-frequency piezoelectric transducers. *Phys.Med. Biol.,* 2016, vol. 61, 2926-2946
- **DE KAMIMURA et al.** Feedback control of microbubble cavitation for ultrasound-mediated blood-brain barrier disruption in non human primates under magnetic résonance guidance. *J. Cereb. Blood Flow Metab.,* 2018, vol. 1 (271678X17753514